# EUROPEAN PATENT SPECIFICATION

(11) **EP 1 012 304 B1**
(45) Date of publication and mention of the grant of the patent: **13.06.2007**
(21) Application number: 98907054.5
(22) Date of filing: 25.02.1998
(51) Int. Cl.: C12N 15/57, C12N 9/50, A61K 38/48, A61K 39/39

(54) **COMPONENT OF BROMELAIN**
BESTANDTEIL VON BROMELAIN
COMPOSANT DE LA BROMELINE

(30) Priority: 25.02.1997 GB 9703827; 25.02.1997 GB 9703850; 28.02.1997 GB 9704252; 25.03.1997 GB 9706119
(43) Date of publication of application: 28.06.2000
(73) Proprietor: Sarantis Pty Ltd, Stones Corner, QLD 4120 (AU)
(72) Inventor: MYNOTT, Tracey, L. Imp. Col. of Sci.Tec.&Medicine, London SW7 2AZ (GB); ENGWERDA, Christian London Sc. Hygiene & Trop. Med, London WC1E 7HT (GB); PEEK, Keith, Cortecs (UK) Lim. Res. & Dev. Div., Deeside, Clwyd CH5 2NT (GB)
(74) Representative: Sutcliffe, Nicholas Robert
(86) International application number: PCT/GB1998/000591
(87) International publication number: WO 1998/038319

(56) References cited:
- WO-A-96/00082
- HARRACH T ET AL: "Isolation and partial characterization of basic proteinases from stem bromelain." JOURNAL OF PROTEIN CHEMISTRY, vol. 14, no. 1, January 1995, pages 41-52, XP002069063
- MUNZIG E ET AL: "Bromelain protease F9 reduces the CD44 mediated adhesionof human peropheral blood lymphocytes to human umbilical vein endothelial cells" FEBS LETTERS, vol. 351, 1994, pages 215-218, XP002069064
- NAPPER ET AL BIOCHEM. J. vol. 301, 1994, pages 727 - 735

## Description

The present invention relates to a fraction of bromelain. In particular, the invention relates to a fraction of bromelain which comprises a novel protein and which has immunomodulatory and anti-tumour activity.

Stem bromelain (bromelain) is the collective name for the proteolytic enzymes found in the tissues of the plant *Bromeliaceae.* It is a mixture of various moieties derived from the stem of the pineapple plant *(Ananas comosus).* Bromelain is known to contain at least five proteolytic enzymes but also non-proteolytic enzymes, including an acid phosphatase and a peroxidase; it may also contain amylase and cellulase activity. In addition, various other components are present.

Bromelain has previously been used in the treatment of a variety of conditions including inflammation and, in particular, it has been used in the treatment of diarrhoea. The use of bromelain in the treatment of infectious diarrhoea is described in WO-A-9301800, where it is suggested that bromelain works by destroying intestinal receptors for pathogens by proteolysis, and in WO-A-8801506, which teaches that bromelain detaches pathogens from intestinal receptors.

Taussig et al, Planta Medica, 1985, 538-539 and Maurer et al, Planta Medica, 1988, 377-381 both suggest that bromelain may be of use in inhibiting tumour growth. US 5,223,406, DE-A-4302060 and JP-A-59225122 also teach the use of bromelain in the treatment of cancer. US 5,223,406 teaches that bromelain is capable of inducing tumour necrosis factor (TNF) while DE-A-4302060 teaches that bromelain can prevent metastasis by the structural modification of the tumour surface protein CD44.

In WO-A-9400147, various experiments were described which demonstrate that proteolytic enzymes and, in particular, bromelain, are capable of inhibiting secretion. The application also discloses that bromelain can reduce toxin binding activity and can inhibit the secretory effect of toxins such as heat labile toxin (LT) and cholera toxin (CT) and also toxins such as heat stable toxin (ST). This is in spite of the fact that ST has a very different mode of action from LT and CT. These observations were explained by the fact that one component of the bromelain mixture, stem bromelain protease, appears to be capable of modulating cyclic nucleotide pathways and this is discussed further in WO-A-9500169. In addition, bromelain has also been demonstrated to inhibit secretion caused by the calcium dependent pathway.

WO-A-9600082 also relates to bromelain and discloses that crude bromelain is capable of interfering with signalling pathways which are important for growth, in particular, signalling pathways which lead to the production of growth factors such as interleukin-2 (IL-2), platelet derived growth factor (PDGF) and insulin like growth factor (IGF). This document teaches that, as a consequence of its ability to block signalling pathways, bromelain is capable of acting as an anti-cancer agent. In addition, bromelain can be used either as an immunosuppressive agent or an immunostimulant depending on the type of cell being treated and whether the cell has previously been activated.

From the prior art, it is clear that bromelain is a mixture which has a variety of different physiological effects. Not all of the components of the bromelain mixture have been characterised and so, except for stem bromelain protease, whose activity we have described, it is not clear which of the components is responsible for which of the various different effects of bromelain. This is, of course, a major disadvantage if the bromelain mixture is to be administered as a pharmaceutical because while one component of bromelain might give the desired effect, there may well be unwanted side effects arising from the action of some other component of the bromelain mixture.

It would therefore be beneficial if individual components of bromelain giving rise to particular medicinal activities could be isolated and administered separately so as to lessen the possibility of side effects.

The present invention relates to one particular fraction which has been isolated from the bromelain mixture and which appears to be at least partially responsible for the immunomodulatory and anti-cancer activity.

The fraction described herein may be isolated from the bromelain mixture by conventional methods, for example by chromatography. High performance liquid chromatograpy (HPLC) is suitable for the purpose and particularly good separation of bromelain proteins may be achieved by fast protein liquid chromatography (FPLC^{™}) using a column packing material such as SP-sepharose. As will be described in more detail in the examples, in chromatography on SP-sepharose using a linear gradient of 0 to 0.8M sodium chloride in acetate buffer over 300ml, the protein of the present invention was the third peak off the column, appearing on the ascending edge of the first main stem bromelain protease peak (the fourth peak off the column).

There is described herein a fraction of bromelain which has a molecular weight of about 27.45 kDa as determined by SDS-PAGE and is obtainable by the following method:
i. dissolving bromelain in 20mM acetate buffer at pH 5.0 containing 0.1mM EDTA sodium;
ii. separating the components of the bromelain by fast protein liquid chromatography on SP-sepharose HP, eluting with a linear gradient of 0 to 0.8 M sodium chloride in acetate buffer over 300 ml; and
iii. collecting the fraction corresponding to the third peak off the column, appearing on the ascending edge of the first main stem bromelain protease peak.

The bromelain fraction described herein, designated CCZ by the inventors, is largely responsible for the immunostimulant activity of bromelain since other known components such as stem bromelain protease (SBP), comosain and ananain have been found to have negligible immunostimmulant activity and, indeed, may act as immunosuppressants. A bromelain fraction designated CCU by the present inventors, has been found by the inventors to have some immunomodulatory activity as has F9, also contained in bromelain (Garbin et al, Int. J. of Oncology, (1994) 5, 197-203).

Also, because it has a much lower number of components than crude bromelain, the bromelain fraction described herein is unlikely to have so many side effects and when used as a pharmaceutical agent and its activity can be more clearly defined.

The present inventors have characterised and obtained the amino acid sequence of the main component of the CCZ fraction. This protein is thought to be responsible for the biological activity of the CCZ fraction.

Therefore, in an aspect of the present invention there is provided a protein as defined in the claims which is a component of bromelain, has a molecular weight of about 27.45 kDa as determined by SDS-PAGE, has an isoelectric point of 9.7 as determined by isoelectric focussing and has the amino terminal sequence:
Val Leu Pro Asp Ser Ile Asp Trp Arg Gln Lys Gly Ala Val Thr Glu Val Lys Asn Arg Gly.

The CCZ fraction described herein is capable of acting as an immunostimulant and as an anti-cancer agent and it is thought that the protein of the invention may be responsible for these activities. It is known from, for example, WO-A-9301800, that the bromelain mixture is a non-specific immunostimulant but it has previously been assumed that one of the known components of bromelain, probably stem bromelain protease, was responsible for this activity. It has now been found that this is not the case and that the protein of the present invention is an immunostimulant whereas stem bromelain protease has neglible immunostimulant activity.

In a further aspect of the invention, there is provided a nucleic acid having a sequence encoding the protein of the invention, or which a sequence complementary thereto. The sequence of such nucleic acids can be determined using standard methods.

In a further aspect of the present invention there is provided a protein according to the invention for use in human or veterinary medicine.

One use of the CCZ fraction described herein is as an immunostimulant and, therefore, there is also provided the use of the protein of the invention in the preparation of an immunostimulant.

Thus the CCZ fraction or the purified protein which is its major component may be used in a method for the treatment of conditions in which the immune system is depressed, the method comprising administering to a patient an effective amount of the CCZ fraction described herein or the protein of the invention.

Because of its ability to stimulate immune responses, the CCZ fraction of bromelain has considerable potential for use in a number of clinical situations where patients are immunocompromised. Primary immunodeficiencies result from genetic abnormalities, while secondary immunodeficiency may arise as a result of malnutrition, infection (for example HIV and malaria), tumours (for example lymphoid, myeloma and other), trauma (for example bums, wounds and surgery), medical treatment (for example with drugs such as steroids, cyclosporin and cyclophospbarwde), protein loss (such as in diarrhoea and bums), diabetes and old age.

Immunodeficiency causes increased susceptibility to a range of viral, protozoal, bacterial and fungal infections and it is associated with many deaths each year as well as being responsible for a substantial proportion of health spending in most countries.

The immune response has two functional divisions: the innate immune system and the adaptive immune system. When a pathogen invades the body, both the adaptive immune response and the innate immune response are activated. Innate immunity provides the first line of defence against infectious agents and most potential pathogens before they establish infection. During this initial phase of innate immunity, the adaptive immune response is developing. If the first defences are breached, the adaptive immune system should be sufficiently developed to produce a specific reaction to the infectious agent, which normally eradicates this agent.

The bromelain fraction described herein may act as an immunomodulator by stimulating both adaptive and innate immunity.

Firstly, it enhances adaptive immunity by increasing T cell activation and increasing antibody production by B cells. Increased T-cell activation would lead to increased production of cytokines such as IL-2, IL-3, IL-4, interferon-γ (IFN-γ), granulocyte-macrophage colony stimulting factor (GM-CSF) IL-1, IL-5, IL-6, IL-10, transforming growth factor-β (TGF-β) and TNF-α. In addition, the protein may promote innate immune response mediated by macrophages, natural killer (NK) cells and neutrophils during the time it takes for the adaptive pathogen-specific immune response to develop.

Stimulation of the innate immune response enables the CCZ fraction of bromelain to be used in situations where either the T or B cell response is not fully functional. This may occur in many secondary immunodeficiencies such as those mentioned above as well as in conditions caused by genetic abnormalities such as those found in severe combined immune deficiency patients who lack functional T and/or B cells.

The bromelain fraction described herein may also promote T cell subset differentiation and compensate for selective T cell subset deficiency.

T cells are divided into two subpopulations, CD4⁺ or "helper" T cells which promote both antibody and cell-mediated immune responses and CD8⁺ or "cytotoxic" T cells which kill cells. Both groups of T cells are essential for host defence against a number of pathogens and a reduction in either T cell population can have devastating effects. This is illustrated by the high susceptibility of AIDS patients, who have depleted CD4⁺ T cell numbers, to opportunistic infections.

The ability of the CCZ fraction to stimulate one T cell population in the absence of the other means that it may be used to treat patients with conditions in which one subpopulation of T cells is depleted; for example patients with bare lymphocyte syndrome (lack functional CD8⁺ cells) or patients with MHC class I deficiency (lack functional CD4⁺ cells) or with various secondary immunodeficiencies such as occur in AIDS patients.

Following stimulation, CD4⁺ T cells can differentiate into either Th1 cells which participate in cell mediated immune responses and Th2 cells which are involved in the generation of humoral (antibody) mediated responses. The protein of the invention is known to stimulate T cells and may selectively affect the development of Th1 and/or Th2 cells. It is therefore likely to stimulate responses mediated by cytokines produced by Th1 cells, for example IL-2, IL-3, IFN-γ, TNF and GM-CSF and/or cytokines produced by Th2 cells, for example IL-4, IL-5, IL-6, IL-10 and TGF-β.

Finally the CCZ fraction also has potential to stimulate T cell-mediated immune responses and therefore to act as a vaccine adjuvant.

In addition to its use as an immunostimmulant, the present inventors have shown that the CCZ fraction is capable of increasing interferon-γ-mediated nitric oxide (NO) production. Thus, the CCZ fraction of bromelain is may be used to treat diseases or conditions which respond to increased NO production.

NO and its derivatives have potent anti-microbial activity against many pathogens including fungi, bacteria and viruses. These include parasites such as *baesia, Brugia, Cryptosporidium, Encephalitoxoon, entamoeba, Leishmania, Naegleria, Ochocerca, Opisthorchis, Plasmodium, Schistosoma, Toxoplasma* and *Trypanosom.* Bacteria affected by NO inlcude *Bacillus, Brucella, Burkholderia, Clostridium, Ehrlichia, Francisella. Klebsiella, Legionella, Listeria, Micrococcus. Pseudomonas Rickettsia, Salmonella. Staphylococcus, Yersinia, Chlamydia* especially C. *trachomatis* and mycobacteria such as *M. avium, M. leprae* and *M. tuberculosis.* NO has activity against fungi such as *Aspergillus, Candida, Cryptococcus, Histoplasma, Pneumocystis* and *Saccharomyces* and against viruses, for example, Coxsackievirus, Ectomelia virus, Encephalomyocarditis virus. Epstein-Barr virus, Herpes simplex virus, Human immunodeficiency virus type 1, Japanese encephalitis virus, mouse hepatitis virus, parvovirus, poliovirus, rabies virus, simian virus 40, vaccinia virus and vesicular stomatitus virus.

The activity of the CCZ fraction in increasing NO production compliments its immunostimmulant activity and means that CCZ can be used as an antimicrobial agent against parasites, bacteria, fungi and viruses such as those listed above.

Thus, in a further aspect of the invention, there is provided the use of the protein of the invention in the preparation of an antimicrobial agent.

The present inventors have also found that the CCZ fraction of bromelain has anti-cancer activity. Various publications have also linked NO production to anti-tumour activity. For example, Hibbs (1991, Res. Immunol. 142, 565-569) has shown that when macrophages produce NO, they kill tumour cells *in vitro.* Thus, increased NO production may be the mechanism by which the CCZ fraction acts against tumours. However, the effectiveness of CCZ as an anti-tumour agent is not dependent upon the correctness of this proposition.

The invention provides, in a further aspect, the use of the protein of the invention in the preparation of an anti-cancer agent.

The CCZ fraction or the protein may be used to treat either solid or soft tumours or other cancers. Examples of cancers which can be treated using the protein of the invention include cancers of the ovary, breast, colon or lung, melanomas, leukaemia and lymphomas. In addition, the protein may be administered to patients receiving cancer chemotherapy to protect against opportunistic infections.

The CCZ fraction or the protein will usually be formulated before administration to patients and so, in a further aspect of the invention there is provided a pharmaceutical or veterinary composition comprising the protein of the invention together with a pharmaceutically or veterinarily acceptable excipient.

In addition to its use as a pharmaceutical agent in its own right, the CCZ fraction or the protein which is its major component may be used as a vaccine adjuvant since it stimulates the immune system to produce a greater number of antibodies after the vaccine is administered.

When used as an adjuvant, the CCZ fraction may be administered separately, either before or after the vaccine. Alternatively, it may be included in a vaccine composition.

Therefore, in another aspect, the invention provides a vaccine composition comprising a vaccine, the protein of the invention as adjuvant and a pharmaceutically or veterinarily acceptable excipient or carrier.

The CCZ fraction of bromelain may be used as an adjuvant in either human or animal vaccines. The vaccine may be a vaccine against a disease, for example a disease caused by a viral, bacterial, fungal or protozoal agent or a disease such as a cancer. Alternatively, the vaccine may be an agent designed to raise antibodies for some other purpose, for example in laboratory animals. The protein may also be used in treatments designed to tolerise the host system against self antigens, for example in autoimmunity.

The CCZ fraction or the protein may be administered by a variety of routes including enteral, for example oral, nasal, buccal, topical or anal administration or parenteral administration, for example by the intravenous, subcutaneous, intramuscular or intraperitoneal routes.

In many cases, the oral route may be preferred as this is often the route which patients find most acceptable. The oral route may be particularly useful if many doses of the protein are required, for example when CCZ is being used to treat a condition in which the immune system is depressed.

When oral adminstration is chosen, it may be desirable to formulate the CCZ fraction or protein in an enteric coated preparation in order to assist its survival through the stomach. Alternatively, another orally administrable dosage form may be used, for example a syrup, elixir or a hard or soft gelatin capsule, either of which may be enteric coated.

However, if it is intended to administer only a single dose, for example when the CCZ fraction or protein is used as a vaccine adjuvant then it may more convenient to use a parenteral route.

For parenteral adminstration, the CCZ fraction or protein may be formulated in distilled water or another pharmaceutically acceptable solvent or suspending agent.

A suitable dose of the CCZ fraction or protein to be administered to a patient may be determined by the clinician. However, as a guide, a suitable dose may be from about 0.5 to 20 mg per kg of body weight. It is expected that in most cases, the dose will be from about 1 to 15 mg per kg of body weight and preferably from 1 to 10 mg per kg of body weight. For a man having a weight of about 70 kg, a typical dose would therefore be from about 70 to 700 mg.

The invention will now be further described with reference to the following examples and to the drawings in which:
FIGURE 1 is an ultra violet elution profile of crude bromelain after cation exchange chromatography on SP Sepharose high performance media;
FIGURE 2 is a plot showing the proteolytic activity and the protein content of crude bromelain fractions after cation exchange chromatography on SP Sepharose high performance media;
FIGURE 3 is an SDS-PAGE of SP Sepharose high performance chromatography pooled fractions run on 4-20 % T gradient gels with lanes 1 to 4 and 6 to 9 containing fractions CCT, CCV, CCX and CCZ and CCY, CCW, CCU and CCS respectively and lanes 5 and 10 containing molecular weight markers;
FIGURE 4 shows isoelectric focussing of pooled fractions run on pH 3-11 gradient gels with Lanes 1, 11 and 12 showing high IEF markers, Lanes 2 and 13 showing crude bromelain and Lanes 3 to 10 showing fractions CCT, CCV, CCX, CCZ, CCY, CCW, CCU and CCS respectively; and
FIGURE 5 is a plot showing the comparative immunomodulatory activity of the fractions obtained from HPLC of crude bromelain. CCZ and CCU increase B cell responses to T cell-dependent antigens, and therefore enhance adaptive immunity.
FIGURE 6 shows the ability of CCZ to increase IFN-γ-mediated nitrite production by macrophages and thus stimulate innate immunity.
FIGURE 7 is a series of plots showing the effect of stem bromelain protease (SBP) and CCZ on the growth inhibition of tumour cells *in vitro.* Results are presented by equivalent protein amount.
FIGURE 8 is a series of plots similar to those in Figure 7 but in which the data has been transformed to represent equivalent proteolytic activity of stem bromelain protease and CCZ.
FIGURE 9 is a comparison of growth inhibitory activity of stem bromelain protease and CCZ against CH1 ovarian tumour growth *in vitro.*

### EXAMPLE 1 - Purification of Bromelain Proteins

### a. Materials

Bromelain was obtained from Solvay Enzymes Inc. (Germany). Fast Flow S Sepharose, Pharmalyte 3-10^{™}, Ampholine 9-11^{™}, Ready Mix IEF^{™} (acrylamide, bisacrylamide) and IEF^{™} markers were obtained from Pharmacia Biotech. Precast 4-20% acrylamide gels and broad range molecular weight markers were obtained from Bio-Rad Laboratories. All other reagents were of analytical grade and obtained from either Sigma Chemical Co. or British Drug House.

### b. Proteinase Assay

The proteolytic activity of bromelain was determined by use of an in-house microtitre plate based assay using the synthetic substrate Z-Arg-Arg-pNA. This assay was based on that described by Filippova et al in Anal. Biochem., 143, 293-297 (1984). The substrate was Z-Arg-Arg-pNA as described by Napper et al in Biochem. J., 301, 727-735, (1994).

### c. Protein Assay

Protein was measured using a kit supplied by Bio-Rad that is a modified method of Lowry et al (J Biol. Chem. (1951) 193, 265-275). Samples were compared to bovine serum albumin standards (0 to 1.5 mg/ml) prepared in either 0.9 % saline or 20 mM acetate buffer pH 5.0, as appropriate.

### d. Preparation of Bromelain

All the following steps were performed at ambient temperature (20 to 25°C). A solution of bromelain (30 mg/ml) was prepared by dissolving 450 mg of powder in 15 ml of 20 mM acetate buffer (pH 5.0) containing 0.1 mM EDTA, sodium. The solution was dispensed into 10 x 1.5 ml microcentrifuge tubes and centrifuged at 13,000 x g for 10 minutes to remove insoluble material. The clear supernatants were pooled and used for chromatography

### e. SP-Sepharose High Performance Chromatography

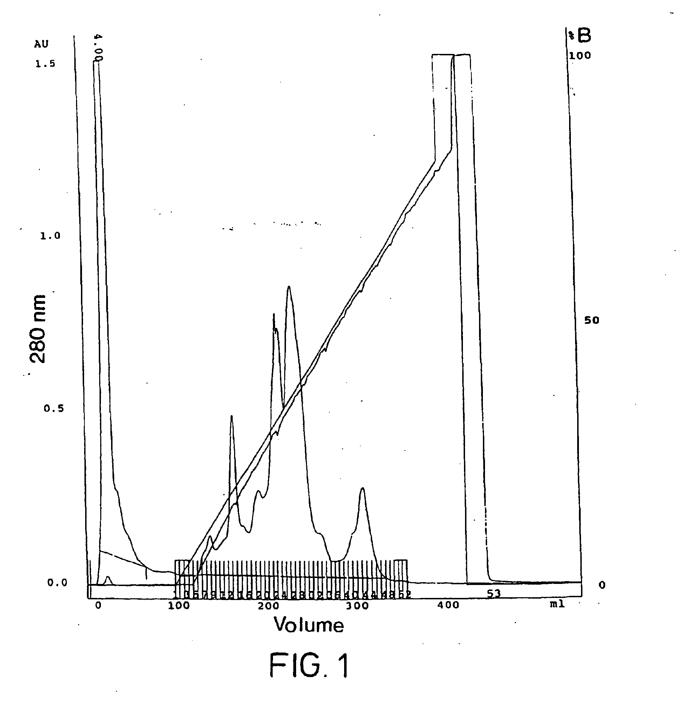

A SP-sepharose column was prepared by packing 25 ml of media into an XK 16/20^{™} column (Pharmacia Biotech) and equilibrated with 20 mM acetate buffer (pH 5.0) containing 0.1 mM EDTA on an FPLC^{™} system at 3ml/min. 5 ml of bromelain solution was injected onto the column. Unbound protein was collected and the column washed with 100 ml of acetate buffer. Protein bound to the column was eluted with a linear gradient of 0 to 0.8 M NaCl in acetate buffer over 300 ml. 5 ml fractions were collected throughout the gradient and Figure 1 shows a typical U.V. chromatogram of crude bromelain obtained from this procedure.

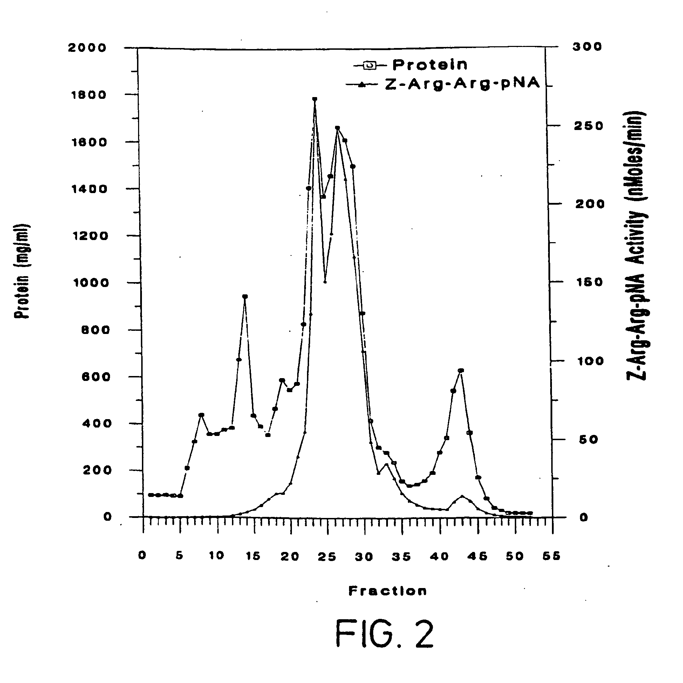

The fractions were then analysed for protein and proteolytic activity as described above and Figure 2 shows the proteolytic activity against the synthetic peptide Z-Arg-Arg-pNA and the protein content of the individual fractions. The protein content profile closely mirrors that of the U.V., as expected, but the main proteolytic activity is confined to the two major peaks that correspond to that of bromelain proteinase (SBP). Small activities are observed in other areas of the chromatogram that may corresponds to other proteinases distinct from bromelain, such as the later eluting Ananain and Comasain (CCS).

The main peaks identified from the U.V. profile were pooled from three successive runs and named as indicated in Table 1. Pooled fractions were used for physicochemical characterisation. Pooled fractions were concentrated by ultrafiltration and buffer exchanged using PD10 columns into isotonic saline (0.9 % w/v NaCl). The protein content and Z-Arg-Arg-pNA activity were calculated prior to biological testing and are shown in Table 2.

The pooled fractions were processed for analysis as described below.

**Table 1. Summary of Pooled Fractions from SP Sepharose HP Fractionated Bromelain (QC2322)**

| **Component** | **Description** | **Fractions Pooled (Inclusive)** |
|---|---|---|
| CCT | Flow through (unbound components) | Unbound column flow through |
| CCV | First peak off column | 8-9 |
| CCX | Second sharp peak off column | 13-14 |
| CCZ | Small peak on ascending edge of the third main bromelain peak | 19-20 |
| CCY | First main bromelain peak | 23-24 |
| CCW | Second main bromelain peak | 27-29 |
| CCU | Small peak on descending edge of the second main bromelain peak | 33-34 |
| CCS | Last double peak off column | 39-44 |

**Table 2. Calculated Protein Content and Z-Arg-Arg-pNA Activity of Pooled Fractions used for Testing Biological Activity.**

| **Pooled Fractions** | **Z-Arg-Arg-pNA Activity (µMoles/min/ml)** | **Protein Content (mg/ml)** |
|---|---|---|
| CCT | 11.30 | 1.00 |
| CCV | 9.78 | 1.00 |
| CCX | 71.71 | 1.00 |
| CCZ | 688.81 | 1.00 |
| CCY | 1500.0 | 0.574 |
| CCW | 1500.0 | 0.543 |
| CCU | 1500.0 | 0.421 |
| CCS | 379.76 | 1.00 |

### f. Processing of Pooled Fractions

The proteolytic activity and protein content of pooled fractions were determined and the concentrations adjusted to approximately either 1.4 mg/ml of protein or 105 nmoles/min/ml of proteinase activity using a Filtron^{™} stirred cell containing an ultrafiltration membrane of nominal molecular weight cut-off of 10 kDa. The fractions were then buffer exchanged using PD10^{™} columns (Pharmacia Biotech) into isotonic saline (0.9 % w/v NaCl), sterile filtered (0.2 µm) and adjusted for protein content or proteolytic activity. Samples were then frozen at -20°C and tested for immunomodulatory activity by the Jerne Hemolytic Plaque Assay.

### g. Sodium Dodecyl Sulphate Polyacrylamide Gel Electrophoresis (SDS-PAGE)

Pooled FPLC^{™} samples were analysed by sodium dodecyl sulphate polyacrylamide gel electrophoresis (SDS-PAGE) on precast 4 to 20 % T gradient gels. Samples were prepared for electrophoresis by acid precipitation in which 100 µl was mixed with an equal volume of 20 % w/v trichloroacetic acid (TCA). Precipitated protein was collected by centrifugation at 13,000 x g for 10 minutes and the supernatant discarded. The pellet was washed twice with 0.5 ml of diethyl ether and left to dry in air at ambient temperature. The pellets were then dissolved in 300 µl of SDS-PAGE sample buffer (62.5 mM Tris-HCl pH 6.8 containing 10 % v/v glycerol, 2 % w/v sodium dodecyl sulphate and 40 mM dithiothreitol) and heated at 95°C in a water bath.

SDS-PAGE broad range molecular weight standards diluted 1:20 in SDS-PAGE sample buffer were treated similarly and run with the samples. Gels were run on a mini Protean II^{™} electrophoresis system according to Bio-Rad's protocol at 240 V and until the dye front reached the end of the gel (30 to 45 min).

After electrophoresis, separated proteins were stained overnight with orbital mixing in a solution of 0.075 % w/v colloidal brilliant blue G-250 containing 1.5 % v/v phosphoric acid, 11.25% w/v ammonium sulphate and 25 % v/v methanol. Gels were destained, to obtain a clear background, in a solution of 25 % v/v methanol and 10 % v/v acetic acid.

### Results

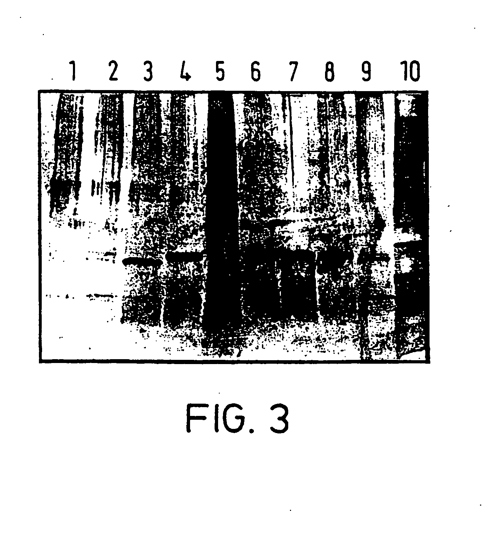

The purity of fractions is shown by SDS-PAGE in Figure 3. All of the pooled fractions except the column flow through (CCT) showed that the major protein present was of molecular weight between approximately 25-28 kDa. This corresponds to the molecular weight of cysteine proteinases isolated from bromelain by other authors (Rowan et al, Methods in Enzymology, (1994), 244, 555-568). The purity of fractions CCX, CCZ, CCY and CCW appears to be high. Minor components of lower molecular weight can be observed in some fractions, particularly CCT, CCV, CCX and CCS. Pooled fractions CCU and CCS contain a doublet between 25-28 kDa; the higher gel loadings of fractions CCX, CCZ, CCY, and CCW means that doublet bands may also be present in these fractions. A summary of the components and their calculated molecular weights in pooled fractions, as determined by SDS-PAGE, is shown in Table 3.

Proteins in pooled fractions CCX, CCZ, CCY+CCW and CCU were transferred onto nitro-cellulose after SDS-PAGE by Western blotting and probed with rabbit antisera raised against purified stem bromelain protease (SBP) (results not shown). All protein bands in these pooled fractions were recognised by antibodies in the sera, indicating immunologically similar proteins, probably belonging to the cysteine proteinase family of enzymes.

**Table 3. Summary of the Molecular Weights of Proteins found in SP Sepharose HP Pooled Fractions as Determined by SDS-PAGE.**

| **Pooled Fractions** | **Molecular Weight (kDa) of Major Protein Band(s)** | **Molecular Weight (kDa) of Minor Protein Band(s)** |
|---|---|---|
| **CCT** | 76.03 | 15.07 |
| **CCV** | 15.07, 25.85, 28.28, 76.03 | |
| **CCX** | 25.08 | 15.07, 76.03 |
| **CCZ** | 27.45 | 13.37, 16.49, 76.03 |
| **CCY** | 27.45 | 6.5 |
| **CCW** | 27.45 | |
| **CCU** | 27.45, 28.28 | |
| **CCS** | 15.07, 25.85, 27.45 | |

### h. Isoelectric Focusing

Pooled fractions (0.5 to 1.0 mg/ml) were diluted 1:3 with deionised water and run on gradient gels of pH 3 to 11. Gels were cast using Ready Mix IEF^{™} to produce a 5.5 % T, 3 % C polyacrylamide gel containing 10 % v/v glycerol, 5.0 % Pharmalyte 3-10^{™} and 2.5 % Ampholine 9-11^{™}. Briefly, 10 µl of sample and high pI markers were loaded onto the gel after prefocusing at 700 V. Sample entry was at 500 V for 10 min, focusing was at 2500 V for 1.5 hour and band sharpening at 3000 V for 10 min. After electrophoresis the proteins were fixed with a solution of 20 % w/v TCA for 30 min, washed in destain for 30 min to remove TCA and stained with brilliant blue G-250 as described for SDS-PAGE (see above).

### Results

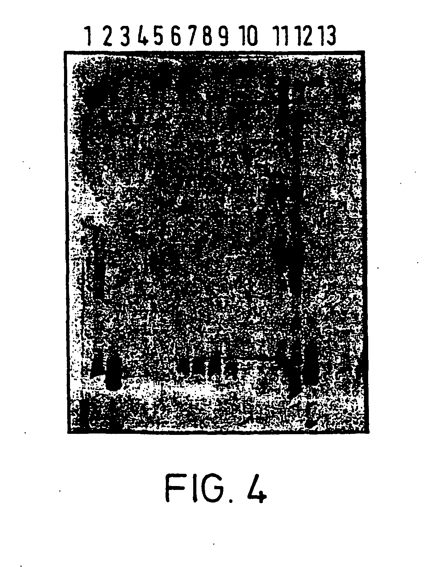

Figure 4 shows that all fractions except CCX contained basic proteins focusing beyond the 9.3 pI marker. Localised charge interactions with the chromatographic media functional groups may explain why proteins of pI 3.8 and 3.85 in CCX, adsorbed onto a cation exchange resin at pH 5.0. CCZ was present as a single band of pI 9.7, whilst pooled fractions CCY, CCW, and CCU contained multiple bands of isoelectric points in the range pH 9.5-9.8. At least part of this heterogeneity can be explained by variation in the carbohydrate moiety of a common stem bromelain protein backbone. The values are in agreement with those reported in the literature of pI 9.45-9.55 for bromelain (Rowan et al, Methods in Enzymology, (1994), 244, 555-568). Pooled fractions CCS contains two basic protein of pI greater than 10.25. Estimates by extrapolation give pIs of 10.4 and 10.45. These correspond to ananain and comasain, and are in agreement with other estimates (Rowan *et al,* as above) of pIs greater than 10. The pIs of proteins in each of the pooled fractions are summarised in Table 4.

**Table 4. Summary of the estimated Isoelectric points of Proteins found in SP Sepharose HP Pooled Fractions.**

| **Pooled** | **Isoelectric Points of Proteins** |
|---|---|
| **Fractions** | |
| **CCT** | Not detected |
| **CCV** | Not Detected |
| **CCX** | 3.8, 3.85 |
| **CCZ** | 9.7 |
| **CCY** | 9.6, 9.7 |
| **CCW** | 9.57, 9.6, 9.7 |
| **CCU** | 9.57, 9.6, 9.75 |
| **CCS** | 10.4, 10.45 |

### i. Western Blotting

Samples run by SDS-PAGE as described above were transferred onto a nitro-cellulose membrane (0.45 µm pore size) using a Transblot^{™} apparatus (Bio-Rad) at 100 V for 1 hour in Towbin buffer as described by the manufacturer. After transfer of protein, the membrane was rinsed in distilled water and then dried in an incubator at 60°C overnight. After drying, the membrane was blocked for 30 min in a 1 % solution of BSA in 20 mM Tris-HCl (pH 7.5) containing 500 mM NaCl (Tris buffer), followed by two 10 min washes in Tris buffer. The membrane was then probed with a 1:50 dilution of anti-bromelain antiserum (rabbit) in Tris buffer, containing 0.05 % v/v Tween 20^{™}, for 2 h. The blot was developed after washing three times in Tris buffer containing Tween 20^{™} and incubating with anti-rabbit horse radish peroxidase for two hours. Immune-reactive bands were visualised by incubation with 4-chloronapthol substrate.

### EXAMPLE 2 - NH₂-terminal Amino Acid Analysis of CCZ Protein

In a separate experiment, pooled fractions of CCZ were run by SDS PAGE and blotted as above onto PVDF membrane. The membrane was stained with 0.025 % w/v coomassie blue R-250, dissolved in 40 % v/v methanol for 10 min, followed by destaining in 50 % v/v methanol. The membrane was dried in air at room temperature and NH₂-terminal amino acid sequencing of the stained proteins was carried out. Briefly, the protein band was cut from the membrane and placed in the upper cartridge of the sequencer. NH₂-terminal amino acid analysis of CCZ protein was determined by Edman degradation using a gas phase sequencer (Applied Biosystems), equipped with an on-line phenylthiohydantion amino acid analyser.

Table 5 shows the first 21 NH₂-terminal amino acids of CCZ and its comparison to the published sequences of stem bromelain protease, ananain and comasain.

**Table 5. NH₂-Terminal Sequence Similarities of CCZ Protein and Those of Known Proteinases Isolated from Bromelain.**

| **Proteinase** | **Position from N-Terminus** |
|---|---|
| **CCZ** | |
| **Stem Bromelain Protease** | |
| **Ananain** | |
| **Comasain** | |

All proteins share sequence homologies. Ananain and comasain differ by 2 out of 20 amino acids when compared stem bromelain protease. In contrast, CCZ differs by 8 out of 21 amino acids when compared to stem bromelain protease. CCZ differs from ananain and comasain by 6 out of 20 amino acids. Whilst it is clear that these proteins are structurally related, CCZ protein is the most distinct, showing significant divergence from the other proteinases isolated from bromelain. CCZ represents a novel protein previously unknown in crude bromelain extracts and thus appears to be a new member of the plant cysteine proteinase family.

### EXAMPLE 3 - Assay of Fractionated Proteins for Immunomodulatory Activity

### a. Materials

Female BALB/c mice were obtained from A. Tuck and Son Ltd. (UK). Mice between 8 and 10 weeks of age were used in all experiments. Sheep red blood cells (SRBC) in Alsevers solution were purchased from TCS Biologicals (Buckingham, UK). Guinea pig complement was purchased from Harlan Sera labs and RPMI 1640 tissue culture media was purchased from Gibco Laboratories. All other reagents were of analytical grade and purchased from Sigma Chemical Co.

### b. Jerne haemolytic plaque assay

The Jerne haemolytic plaque assay (Weir, D. M. (ed.). 1986. Handbook of Experimental Immunology, 1-4, 4th edn. Blackwell Scientific Publications, Oxford, UK) was used to assay adjuvant potential of fractionated proteins. Experiments were conducted on fractionated samples of bromelain which were prepared on two separate occasions. Each sample was given a unique code (Table 1), diafiltered and concentrated, where appropriate into isotonic saline by ultrafiltration (see Example 1(f)). All experiments were conducted in a double-blind manner.

Mice were administered a single intravenous injection of either crude bromelain, fractionated protein or saline as shown in Table 6. Crude bromelain (200pg; 1500 umoles/min/ml) was suspended in 0.9 % (200 µl) saline and filter sterilised immediately prior to administration. Fractionated samples (200 µl) were similarly filter sterilised. Mice administered with sterile saline alone were used as controls. The dose rate for the crude bromelain of 1500 µmoles/min/ml corresponds to approximately one third of LD₅₀ for bromelain as given in the Materials Safety Data Sheet available from Sigma Aldrich Ltd. Following administration of crude bromelain, fractionated proteins or saline, mice were immunised by interperitoneal injections with sheep red blood cells (SRBC) (100 µl;10⁷ cells). Mice used as negative controls were administered with saline alone (100 µl). Mice were sacrificed 3 days following immunisation at which time the spleens were removed and splenocytes isolated by filtration through a nylon mesh filter. The number of B cells producing antibodies specific for SRBC antigen (i.e. plaque forming cells (PFC) per 10⁶ splenocytes) was determined using the Jerne haemolytic plaque assay.

### c. Data Analysis

Numerical values are expressed as the mean ± standard deviation. Independent t-tests (Students t-test; 2-way) were used to test for differences among means in PFCs. An analysis of variance (ANOVA) was used when the mean of more than one treatment was compared with controls.

### d. Results

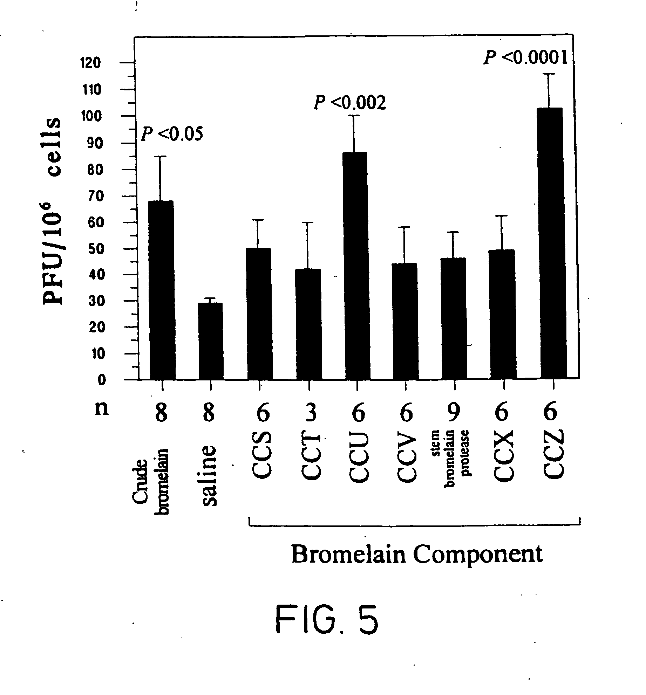

Assay results from the Jerne Haemolytic plaque assay are shown in Figure 5 and in Table 6.

**Table 6 PFC Formation (number of B-cells secreting antibodies to SRBC)**

| *Group* | Treatment | No. of animals | Mean PFC/10⁶ splenocytes | Median (Range) | S.D |
|---|---|---|---|---|---|
| *F* | brom + SRBC | 8 | 68 | 65 (1-147) | 47 |
| *L* | saline + SRBC | 8 | 29 | 28 (21-40) | 7 |
| *S* | CCS + SRBC | 6 | 50 | 47(13-90) | 28 |
| *T* | CCT + SRBC | 3 | 42 | 26(13-86) | 32 |
| *U* | CCU + SRBC | 6 | 86 | 94 (25-123) | 35 |
| *V* | CCV + SRBC | 6 | 44 | 35(3-90) | 34 |
| *W*/*Y* | SBP + SRBC | 9 | 46 | 37 (4-112) | 29 |
| *X* | CCX + SRBC | 6 | 49 | 39 (8-98) | 33 |
| Z | CCZ + SRBC | 6 | 102 | 95(68-106) | 31 |

Administration of crude bromelain (QC2322) caused a significant increase in PFC compared with saline controls (bromelain PFC/10⁶ splenocytes, 68 ± 47; saline control, 29 ± 7; *P* < 0.05). Fractionated proteins CCZ and CCU also induced significantly higher PFC than saline controls (102 ± 31 and 86 ± 31 PFC/10⁶ splenocytes, respectively; *P* < 0.002) and produced more PFC than the crude bromelain extract. Components CCZ and CCU elute differently from the FPLC^{™} column than the well described stem bromelain protease, comosain, ananain and F9 enzymes, suggesting that CCZ and CCU are distinct molecules. When purified stem bromelain protease (combined components CCW and CCY) was tested for its adjuvant effect, results demonstrate only a small increase in PFC (46 ± 29 PFC/10⁶ splenocytes), which suggests that stem bromelain protease is not the component responsible for the adjuvant activity of bromelain. Similarly, ananain and comosain (CCS) also had negligible immunomodulatory activity. Therefore the adjuvant effect of crude bromelain does not appear to be attributed to components in the crude mixture previously considered to have an immunological role. Some components tested had PFC values higher than controls (and similar to stem bromelain protease), however these values were not significantly different. The slight increase in PFC observed with these components and stem bromelain protease is similar to that previously seen when mice were administered trypsin and responses may be attributed to non-specific proteolytic effects.

### EXAMPLE 4 - CCZ Increases Nitrate Production by Macrophages and Threrefore Can Stimulate Innate Immune Response

Example 3 demonstrated that CCZ could increase the adaptive immune response by enhancing B cell responses to T cell-dependent antigens. We next wished to investigate whether CCZ could also enhance innate immunity by investigating its effect on macrophages, the major cell population involved in innate immunity.

An important host defence mechanism against intracellular parasites is the production of nitric oxide (NO) by macrophages. We therefore investigated whether CCZ could affect NO production.

### a. Materials

The murine macrophage cell line RAW 264 was stimulated in culture with recombinant IFN-γ (100 U/ml). Nitrite levels in culture supernatants were measured using the Greiss assay (Roach et al (1991), Infection and Immunity, 59, 3935-3944).

### b. Method

RAW 264 macrophages were treated with either CCZ (50µg/ml), crude bromelain (50µg/ml) or stem bromelain protease (50µg/ml) or mock-treated with saline. Cells were then washed three times to remove the treatment, and then stimulated with IFN-γ.

### c. Results

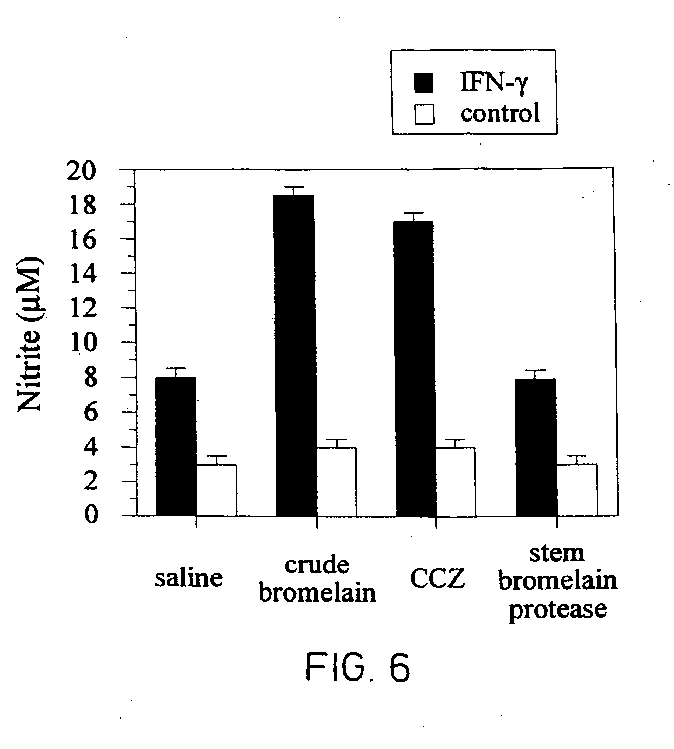

Crude bromelain and CCZ, but not stem bromelain protease, were found to increase significantly IFN-γ-mediated nitrite production (Figure 6). In CCZ treated cells, the increase in IFN-γ-mediated nitrite production was significantly greater than saline-treated cells, suggesting that CCZ synergises with IFN-γ to increase NO production. Negligible nitrite was produced when macrophages were stimulated with CCZ or bromelain alone, indicating that neither CCZ norbromelain activates nitrite production. To ensure that potential contaminating endotoxin, which may have been present in the CCZ mixture, was not responsible for the increase in NO, polymyxin B (a potent inhibitor of endotoxin) was included in experiments. The inclusion of polymyxin B did not affect the IFN-γ-induced increase in NO production by CCZ-treated cells, indicating that potential contaminating endotoxin was not responsible for the observed effect (data not shown).

Given the importance of nitric oxide production for host defence against intracellular pathogens, the ability of CCZ to stimulate specific production of this metabolite by macrophages indicates that it may control various intracellular infections.

### EXAMPLE 5 - In vitro Growth Inhibition of Bromelain Fragments Against a Panel of Human Tumour Cell Lines

In addition to the critical role of NO in host defence against infection, it has also been demonstrated to be a potent killer of tumour cells. Hibbs (1991, Res. Immunol., 142, 565-569) has shown that when macrophages produce NO, they kill tumour cells *in vitro.* Nitric oxide has therefore been proposed to be of use in cancer therapy (Sagar et al, (1995), Cancer Treatment Reviews, 21, 159-181). We therefore investigated whether CCZ could block tumour growth of several different human tumour cell lines *in vitro* and therefore act as an anti-tumour agent. The comparative growth inhibitory properties of CCZ protein and stem bromelain protease was determined against a panel of fifteen human tumour cell lines representative of five of the most common solid cancers in humans: ovarian, colon, breast, lung and melanoma.

Cell lines were trypsinised and single viable cells were seeded into 96-well microtitre plates at a density of 4 x 10³ per well in 160:1 growth medium. After allowing for attachment overnight, CCZ, SBP or bromelain were then added to quadruplicate wells in growth medium to give a range of final concentrations in wells of 50, 10, 2.5, 1 and 0.25:g/ml. Eight wells were allocated as control untreated cells. Extracts were diluted immediately prior to addition to cells in sterile water. Extract exposure was for 96 hours whereupon cell number in each well was determined using staining with 0.4% sulforhodamine B in 1% acetic acid as described previously (Kelland et al, Cancer Res., 53, 2581-2586 (1993)). 50% inhibitory concentrations (IC₅₀ in :g/ml) were then calculated from plots of concentration versus % control absorbance (read at 540nm).

### Results

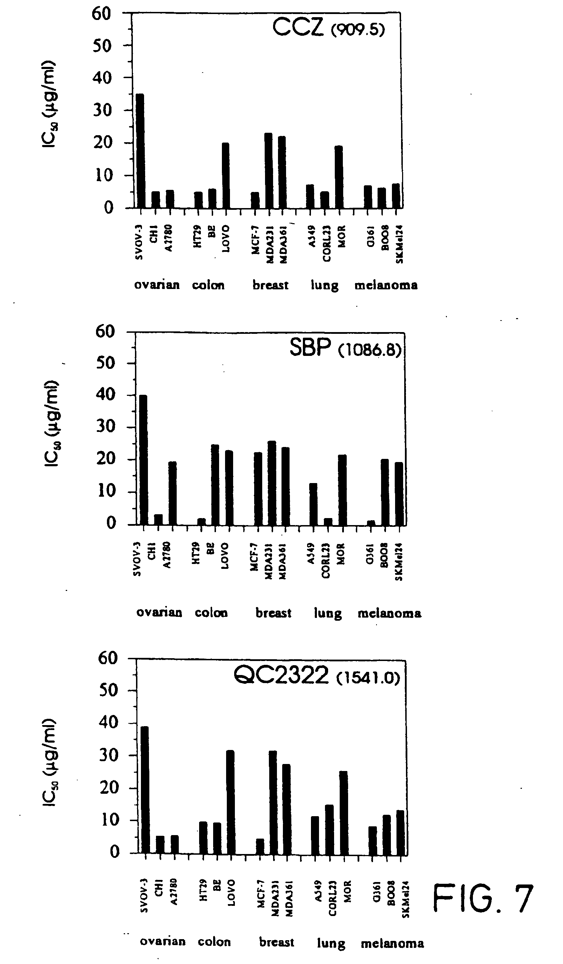

The extracts were successfully dissolved and IC₅₀ values are shown in Figure 7.

As can be seen from Figure 7, CCZ protein showed an *in vitro* potency comparable to that of QC2322 (crude bromelain) and stem bromelain protease against all of the cell lines. CCZ exhibited a mean IC₅₀ across all 15 lines of 11.9 µg/ml, with generally less activity against the SKOV-3 (ovarian), LOVO (colon), MDA231 (breast), MDA361 (breast) and MOR (lung).

The method used in this example relies on the detection of cells immobilised to wells of a microtitre tray. Growth inhibitory activity is determined by staining of cells following treatment with bromelain fractions. Dead or dying cells become detached from the wells and therefore do not stain. Cells may also be removed from wells by treating with high concentrations of enzymes such as trypsin; a process referred to as "trypsinisation". It is therefore possible that the growth inhibitory activity of CCZ and bromelain is caused by a non-specific removal of cells from wells arising from proteolytic activity rather than a specific "anti-cancer" effect of the fractions.

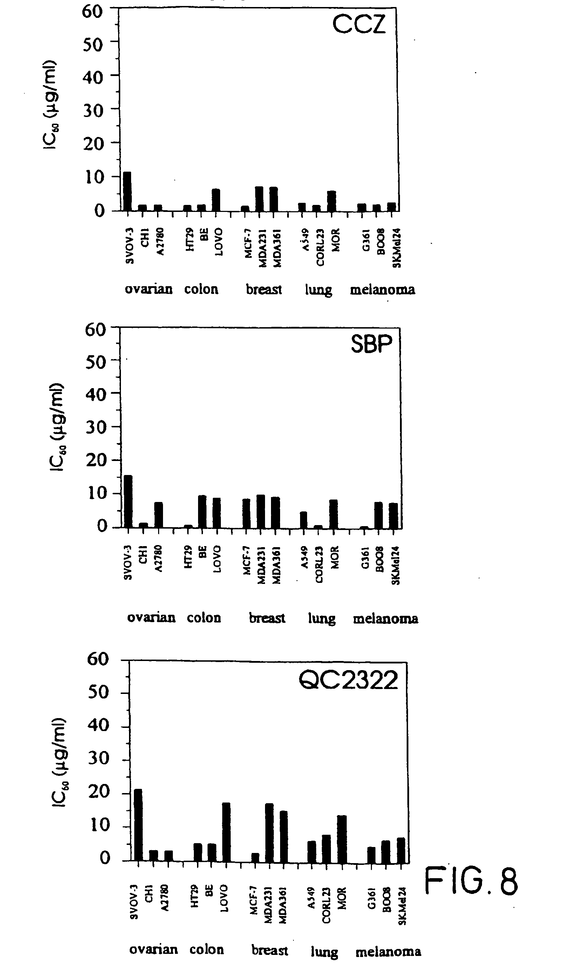

In view of this, the results given in Figure 7 were adjusted for the proteolytic activity of each of the fractions. The adjusted results are given in Figure 8.

Using this interpretation, it can be seen from Figure 8 that a somewhat different analysis of the results is obtained. Once the proteolytic activity of each of the fractions has been taken into account, it can be seen that CCZ has significantly greater anti-cancer activity than either crude bromelain or stem bromelain protease.

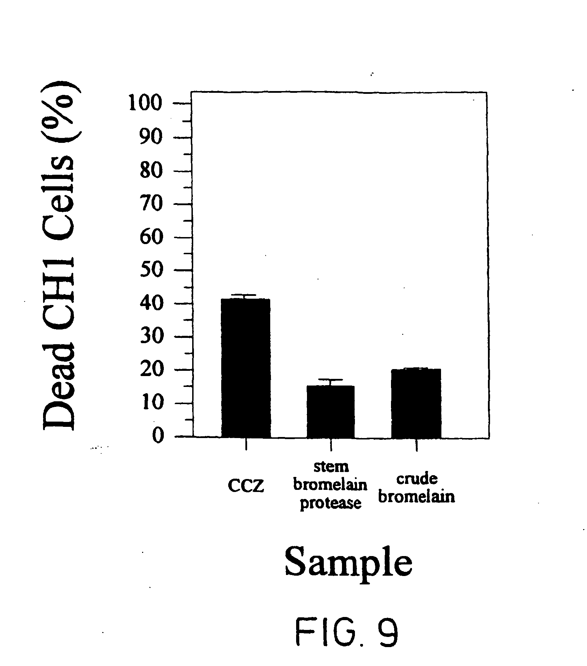

To confirm that CCZ does indeed have growth inhibitory activity, crude bromelain, stem bromelain protease and CCZ were diluted to contain equivalent proteolytic activity (5.7 µMoles/min/ml) and tested for their ability to prevent CH1 ovarian tumour growth. Figure 9 confirms that CCZ does, indeed, have more potent growth inhibitory activity than either crude bromelain or stem bromelain protease.

## Claims

1. An isolated protein which has a molecular weight of about 27.45 kDa as determined by SDS-PAGE, an isoelectric point of 9.7 as determined by isoelectric focussing and an N-terminal amino acid sequence Val Leu Pro Asp Ser Ile Asp Trp Arg Gln Lys Gly Ala Val Thr Glu Val Lys Asn Arg Gly, which isolated protein is a component of bromelain obtainable by the following method:
(i) dissolving bromelain in 20 mM acetate buffer at pH 5.0 containing 0.1 mM EDTA sodium;
(ii) separating the components of the bromelain by fast protein liquid chromatography on SP-sepharose HP, eluting with a linear gradient of 0 to 0.8 M sodium chloride in acetate buffer over 300 mL;
(iii) collecting the fraction corresponding to the third peak off the column, appearing on the ascending edge of the first main stem bromelain protease peak; and
(iv) isolating the protein from the fraction obtained at step (iii).

2. An isolated nucleic acid which encodes the isolated protein of Claim 1.

3. An isolated nucleic acid which is complementary to the isolated nucleic acid of Claim 2.

4. A recombinant protein which has a molecular weight of about 27.45 kDa as determined by SDS-PAGE, an isoelectric point of 9.7 as determined by isoelectric focussing and an N-terminal amino acid sequence Val Leu Pro Asp Ser Ile Asp Trp Arg GIn Lys Gly Ala Val Thr Glu Val Lys Asn Arg Gly, produced using the isolated nucleic acid of Claim 2.

5. The isolated protein of Claim 1 or the recombinant protein of Claim 4 for use in human or veterinary medicine.

6. Use of the isolated protein of Claim 1 or the recombinant protein of Claim 4 in the manufacture of a medicament for human or veterinary medicine.

7. Use of the isolated protein of Claim 1 or the recombinant protein of Claim 4 in the manufacture of an immunostimulatory medicament.

8. Use according to Claim 7, wherein the immunostimulatory medicament is for the treatment of primary immunodeficiencies or secondary immunodeficiencies resulting from malnutrition, infection, tumours, trauma, medical treatment with drugs, protein loss, diabetes and old age.

9. Use according to Claim 7, wherein the immunostimulatory, medicament is a vaccine adjuvant.

10. Use of the isolated protein of Claim 1 or the recombinant protein of Claim 4 in the manufacture of a medicament for treating a microbial infection.

11. Use of the isolated protein of Claim 1 or the recombinant protein of Claim 4 in the in the manufacture of a medicament for treating cancer.

12. Use according to Claim 11 wherein the cancer is ovary, breast, colon or lung cancer, melanoma, leukaemia or lymphoma.

13. A pharmaceutical or veterinary composition comprising the isolated protein of Claim 1 or the recombinant protein of Claim 4 together with a pharmaceutically or veterinarily acceptable excipient or carrier.

14. A vaccine composition comprising a vaccine, an adjuvant comprising the isolated protein of claim 1 or the recombinant protein of Claim 4 together with a pharmaceutically or veterinarily acceptable excipient or carrier.

15. The vaccine composition of Claim 14, which is formulated for enteral, nasal, buccal or anal administration.

16. The vaccine composition of Claim 14, which is formulated for parenteral administration.

17. The vaccine composition of Claim 16, wherein parenteral administration is by an intravenous, subcutaneous, intramuscular or intraperitoneal route.

## Patentansprüche

1. Isoliertes Protein, das ein Molekulargewicht von etwa 27,45 kDa aufweist, wie es durch SDS-PAGE bestimmt ist, einen isoelektrischen Punkt von 9,7, wie er durch isoelektrische Fokussierung bestimmt ist, und eine N-terminale Aminosäuresequenz Val Leu Pro Asp Ser Ile Asp Trp Arg Gln Lys Gly Ala Val Thr Glu Val Lys Asn Arg Gly, welches isolierte Protein ein Bestandteil Bromelain ist, das durch das folgende Verfahren erhältlich ist:
(i) Auflösen von Bromelain in 20 mM Acetatpuffer bei pH 5,0, der 0,1 mM Natrium-EDTA enthält;
(ii) Trennen der Bestandteile des Bromelains durch schnelle Protein-Flüssigkeitschromatographie auf SP-Sepharose HP, Eluieren mit einem linearen Gradienten von 0 bis 0,8 M Natriumchlorid in Acetatpuffer über 300 mL;
(iii) Sammeln der Fraktion, die dem dritten Peak aus der Säule entspricht, der auf der ansteigenden Kante der ersten Hauptlinie des Bromelain-Protease-Spitzenwerts auftritt; und
(iv) Isolieren des Proteins von der in Schritt (iii) erhaltenen Fraktion.

2. Isolierte Nukleinsäure, die für das isolierte Protein nach Anspruch 1 kodiert.

3. Isolierte Nukleinsäure, die zu der isolierten Nukleinsäure von Anspruch 2 komplementär ist.

4. Rekombinantes Protein, das ein Molekulargewicht von etwa 27,45 kDa aufweist, wie es durch SDS-PAGE bestimmt ist, einen isoelektrischen Punkt von 9,7, wie er durch isoelektrisches Fokussieren bestimmt ist, und eine N-terminale Aminosäuresequenz Val Leu Pro Asp Ser Ile Asp Trp Arg Gln Lys Gly Ala Val Thr Glu Val Lys Asn Arg Gly, hergestellt unter Verwendung der isolierten Nukleinsäure von Anspruch 2.

5. Isoliertes Protein nach Anspruch 1 oder das rekombinante Protein nach Anspruch 4 zur Verwendung in der Human- oder Tiermedizin.

6. Verwendung des isolierten Proteins nach Anspruch 1 oder des rekombinanten Proteins nach Anspruch 4 zur Herstellung eines Medikaments für die Human- oder Tiermedizin.

7. Verwendung des isolierten Proteins nach Anspruch 1 oder des rekombinanten Proteins nach Anspruch 4 bei der Herstellung eines immunstimulatorischen Medikaments.

8. Verwendung gemäß Anspruch 7, bei der das immunstimulatorische Medikament zur Behandlung primärer Immundefizienzen oder sekundärer Immundefizienzen ist, die sich aus Mangelernährung, Infektion, Tumoren, Trauma, medizinischer Behandlung mit Arzneimitteln, Proteinverlust, Diabetes und hohem Alter ergeben.

9. Verwendung nach Anspruch 7, bei der das immunstimulatorische Medikament ein vakzin-Adjuvanz ist.

10. Verwendung des isolierten Proteins nach Anspruch 1 oder des rekombinanten Proteins nach Anspruch 4 zur Herstellung eines Medikaments zur Behandlung einer mikrobiellen Infektion.

11. Verwendung des isolierten Proteins nach Anspruch 1 oder des rekombinanten Proteins nach Anspruch 4 bei der Herstellung eines Medikaments zur Behandlung von Krebs.

12. Verwendung nach Anspruch 11, bei der der Krebs Eierstock-, Brust-, Darm- oder Lungenkrebs ist, ein Melanom, Leukämie oder Lymphom.

13. Pharmazeutische oder veterinärmedizinische Zusammensetzung, die das isolierte Protein nach Anspruch 1 oder das rekombinante Protein nach Anspruch 4 zusammen mit einem pharmazeutisch oder veterinärmedizinisch annehmbaren Vehikulum oder Trägerstoff umfasst.

14. Impfstoffzusammensetzung umfassend ein Vakzin, ein Adjuvanz, umfassend das isolierte Protein von Anspruch 1 oder das rekombinante Protein nach Anspruch 4, zusammen mit einem pharmazeutisch oder veterinärmedizinisch annehmbaren Vehikulum oder Trägerstoff.

15. Impfstoffzusammensetzung nach Anspruch 14, die zur enteralen, nasalen, buccalen oder analen Verabreichung formuliert ist.

16. Impfstoffzusammensetzung nach Anspruch 14, die zur parenteralen Verabreichung formuliert ist.

17. Impfstoffzusammensetzung nach Anspruch 16, bei der die parenterale Verabreichung durch eine intravenöse, subkutane, intramuskuläre oder intraperitoniale Route erfolgt.

## Revendications

1. Protéine isolée ayant une masse moléculaire d'environ 27,45 kDa telle que déterminée par SDS-PAGE, un point isoélectrique de 9,7 tel que déterminé par focalisation isoélectrique et une séquence d'acides aminés N-terminale Val Leu Pro Asp Ser Ile Asp Trp Arg Gln Lys Gly Ala Val Thr Glu Val Lys Asn Arg Gly, laquelle protéine isolée est un composant de la broméline obtenu par le procédé suivant :
(i) dissolution de la broméline dans un tampon acétate 20 mM à pH 5,0 contenant de l'EDTA sodique 0,1 mM ;
(ii) séparation des composants de la broméline par chromatographie en phase liquide rapide des protéines sur SP-Sepharose HP, en éluant avec un gradient linéaire de chlorure de sodium 0 à 0,8 M dans un tampon acétate sur 300 mL ;
(iii) collecte de la fraction correspondant au troisième pic de la colonne, apparaissant sur le bord ascendant du premier pic principal de la protéase broméline de la tige ; et
(iv) isolement de la protéine de la fraction obtenue à l'étape (iii).

2. Acide nucléique isolé codant pour la protéine isolée selon la revendication 1.

3. Acide nucléique isolé complémentaire de l'acide nucléique isolé selon la revendication 2.

4. Protéine recombinante ayant une masse moléculaire d'environ 27,45 kDa telle que déterminée par SDS-PAGE, un point isoélectrique de 9,7 tel que déterminé par focalisation isoélectrique et une séquence d'acides aminés N-terminale Val Leu Pro Asp Ser Ile Asp Trp Arg Gln Lys Gly Ala Val Thr Glu Val Lys Asn Arg Gly, produite en utilisant l'acide nucléique isolé selon la revendication 2.

5. Protéine isolée selon la revendication 1, ou la protéine recombinante selon la revendication 4, destinée à être utilisée en médecine humaine ou vétérinaire.

6. Utilisation de la protéine isolée selon la revendication 1, ou de la protéine recombinante selon la revendication 4, dans la fabrication d'un médicament destiné à la médecine humaine ou vétérinaire.

7. Utilisation de la protéine isolée selon la revendication 1, ou de la protéine recombinante selon la revendication 4, dans la fabrication d'un médicament immunostimulateur.

8. Utilisation selon la revendication 7, dans laquelle le médicament immunostimulateur est destiné au traitement d'immunodéficiences primaires ou d'immunodéficiences secondaires résultant de la malnutrition, d'une infection, de tumeurs, d'un traumatisme, d'un traitement médical avec des médicaments, d'une perte protéique, du diabète et de la vieillesse.

9. Utilisation selon la revendication 7, dans laquelle le médicament immunostimulateur est un adjuvant vaccinal.

10. Utilisation de la protéine isolée selon la revendication 1, ou de la protéine recombinante selon la revendication 4, dans la fabrication d'un médicament destiné au traitement d'une infection microbienne.

11. Utilisation de la protéine isolée selon la revendication 1, ou de la protéine recombinante selon la revendication 4, dans la fabrication d'un médicament destiné au traitement d'un cancer.

12. Utilisation selon la revendication 11, dans laquelle le cancer est un cancer des ovaires, du sein, du côlon ou du poumon, un mélanome, une leucémie ou un lymphome.

13. Composition pharmaceutique ou vétérinaire comprenant la protéine isolée selon la revendication 1, ou la protéine recombinante selon la revendication 4, conjointement avec un vecteur ou un excipient acceptables sur le plan pharmaceutique ou vétérinaire.

14. Composition vaccinale comprenant un vaccin, un adjuvant comprenant la protéine isolée selon la revendication 1, ou la protéine recombinante selon la revendication 4, conjointement avec un vecteur ou un excipient acceptables sur le plan pharmaceutique ou vétérinaire.

15. Composition vaccinale selon la revendication 14, qui est formulée pour une administration entérale, nasale, buccale ou anale.

16. Composition vaccinale selon la revendication 14, qui est formulée pour une administration parentérale.

17. Composition vaccinale selon la revendication 16, dans laquelle l'administration parentérale est réalisée par une voie intraveineuse, sous-cutanée, intramusculaire ou intrapéritonéale.


### SEQUENCE LISTING

(1) GENERAL INFORMATION:
   (i) APPLICANT:
      (A) NAME: CORTECS (UK) LIMITED
      (B) STREET: FIRST FLOOR, OSPREY HOUSE
      (C) CITY: LOWER SQUARE,
      (D) STATE: ISLEWORTH, MIDDLESEX
      (E) COUNTRY: LONDON, ENGLAND
      (F) POSTAL CODE (ZIP): TW7 6BN

      (A) NAME: MYNOTT, Tracey, Lehanne
      (B) STREET: IMPERIAL COLLEGE OF SCIENCE TECHNOLOGY & MEDICINE
      (C) CITY: DEPT OF BIOCHEMISTRY,
      (D) STATE: EXHIBITION RD,
      (E) COUNTRY: LONDON, UNITED KINGDOM
      (F) POSTAL CODE (ZIP): SW7 2AZ

      (A) NAME: ENGWERDA, Christian
      (B) STREET: LONDON SCHOOL OF HYGIENE & TROPICAL MEDICINE
      (C) CITY: DEPT OF MEDICAL PARASITOLOGY
      (D) STATE: KEPPEL STREET,
      (E) COUNTRY: LONDON, UNITED KINGDOM
      (F) POSTAL CODE (ZIP): WCLE 7HT

      (A) NAME: PEEK, Keith
      (B) STREET: CORTECS (UK) LIMITED
      (C) CITY: RESEARCH & DEVELOPMENT DIV
      (D) STATE: NEWTECH SQUARE,
      (E) COUNTRY: DEESIDE IND PARK, UK
      (F) POSTAL CODE (ZIP): CH5 2NT
   (ii) TITLE OF INVENTION: COMPONENT OF BROMELAIN
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: WO PCT/GB98/00591
(2) INFORMATION FOR SEQ ID NO: 1:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 1:
   Val Leu Pro Asp Ser Ile Asp Trp Arg Gln Lys Gly Ala Val Thr Glu
   1               5                   10                  15
   Val Lys Asn Arg Gly
               20
(2) INFORMATION FOR SEQ ID NO: 2:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 2:
   Ala Val Pro Gln Ser Ile Asp Trp Arg Asp Tyr Gly Ala Val Thr Ser
   1               5                   10                  15
   Val Lys Asn Gln Asn
               20
(2) INFORMATION FOR SEQ ID NO: 3:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 3:
   Val Pro Gln Ser Ile Asp Trp Arg Asp Ser Gly Ala Val Thr Ser Val
   1               5                   10                  15
   Lys Asn Gln Gly
               20
(2) INFORMATION FOR SEQ ID NO: 4:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 4:
   Val Pro Gln Ser Ile Asp Trp Arg Asn Tyr Gly Ala Val Thr Ser Val
   1               5                   10                  15
   Lys Asn Gln Gly
               20

### SEQUENCE LISTING

(1) GENERAL INFORMATION:
   (i) APPLICANT:
      (A) NAME: CORTECS (UK) LIMITED
      (B) STREET: FIRST FLOOR, OSPREY HOUSE
      (C) CITY: LOWER SQUARE,
      (D) STATE: ISLEWORTH, MIDDLESEX
      (E) COUNTRY: LONDON, ENGLAND
      (F) POSTAL CODE (ZIP): TW7 6BN

      (A) NAME: MYNOTT, Tracey, Lehanne
      (B) STREET: IMPERIAL COLLEGE OF SCIENCE TECHNOLOGY & MEDICINE
      (C) CITY: DEPT OF BIOCHEMISTRY,
      (D) STATE: EXHIBITION RD,
      (E) COUNTRY: LONDON, UNITED KINGDOM
      (F) POSTAL CODE (ZIP): SW7 2AZ

      (A) NAME: ENGWERDA, Christian
      (B) STREET: LONDON SCHOOL OF HYGIENE & TROPICAL MEDICINE
      (C) CITY: DEPT OF MEDICAL PARASITOLOGY
      (D) STATE: KEPPEL STREET,
      (E) COUNTRY: LONDON, UNITED KINGDOM
      (F) POSTAL CODE (ZIP): WC1E 7HT

      (A) NAME: PEEK, Keith
      (B) STREET: CORTECS (UK) LIMITED
      (C) CITY: RESEARCH & DEVELOPMENT DIV
      (D) STATE: NEWTECH SQUARE,
      (E) COUNTRY: DEESIDE IND PARK, UK
      (F) POSTAL CODE (ZIP): CH5 2NT
   (ii) TITLE OF INVENTION: COMPONENT OF BROMELAIN
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
   (v) CURRENT APPLICATION DATA: APPLICATION NUMBER: WO PCT/GB98/00591
(2) INFORMATION FOR SEQ ID NO: 1:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 1:
   Val Leu Pro Asp Ser Ile Asp Trp Arg Gln Lys Gly Ala Val Thr Glu
   1               5                   10                  15
   Val Lys Asn Arg Gly
               20
(2) INFORMATION FOR SEQ ID NO: 2:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 2:
   Ala Val Pro Gln Ser Ile Asp Trp Arg Asp Tyr Gly Ala Val Thr Ser
   1               5                   10                  15
   Val Lys Asn Gln Asn
               20
(2) INFORMATION FOR SEQ ID NO: 3:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION: SEQ ID NO: 3:
   Val Pro Gln Ser Ile Asp Trp Arg Asp Ser Gly Ala Val Thr Ser Val
   1               5                   10                  15
   Lys Asn Gln Gly
               20
(2) INFORMATION FOR SEQ ID NO: 4:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (xi) SEQUENCE DESCRIPTION : SEQ ID NO: 4:
   Val Pro Gln Ser Ile Asp Trp Arg Asn Tyr Gly Ala Val Thr Ser Val
   1               5                   10                  15
   Lys Asn Gln Gly
               20